# EUROPEAN PATENT APPLICATION

(11) **EP 1 418 523 A2**
(43) Date of publication of application: **12.05.2004**
(21) Application number: 03025631.7
(22) Date of filing: 06.11.2003
(51) Int. Cl.: G06F 19/00

(54) **Diagnosis support system for diabetes**

(30) Priority: 06.11.2002 JP 2002322901
(71) Applicant: Sysmex Corporation, Kobe-shi, Hyogo 651-0073 (JP)
(72) Inventor: Asano, Kaoru, Chuo-ku Kobe-shi Hyogo 651-0073 (JP); Saitou, Takeo, Chuo-ku Kobe-shi Hyogo 651-0073 (JP); Kouchi, Yasuhiro, Chuo-ku Kobe-shi Hyogo 651-0073 (JP); Kishi, Kazuki, Chuo-ku Kobe-shi Hyogo 651-0073 (JP); Nakajima, Hiromu, Osaka-shi, Osaka 545-0014 (JP)
(74) Representative: HOFFMANN - EITLE

(57) **Abstract**

A diagnosis support system for diabetes has: a diagnostic data input unit for entering diagnostic data including the clinical testing data and clinical findings of the patient; a pathophysiologic condition pattern analyzing unit for analyzing the pathophysiologic condition of diabetes of the patient by comparing the diagnostic data and a predetermined criteria of analysis; a diagnosis support information generating unit for generating diagnosis support information based on the diagnostic data and diagnostic criteria of determination determined by each analyzed pathophysiologic condition, and a diagnosis support information output unit for supplying information obtained by the pathophysiologic condition pattern analyzing unit and the diagnostic information generating unit.

## Description

### BACKGROUND OF THE INVENTION

### 1. Field of the Invention

The present invention relates to a diagnosis support system for diabetes and, more particularly, to a diagnosis support system for diabetes for conducting an analysis of pathophysiologic conditions and providing diagnosis support information that is available to a therapeutic method for diabetes.

### 2. Description of the Related Art

Diabetes is one of typical life-style related diseases, and the number of patients is increasing rapidly in association with westernization of our life-style.

According to an "actual condition survey of diabetes" conducted on a national nutrition survey of November 1997 in Japan, it was reported that the number of patients with diabetes reached as many as 13,700,000 including potential patients.

Pathophysiologic conditions of type 2 diabetes are mainly classified into several subtypes from "hepatic glucose production", "insulin secretion ability", "insulin resistance" and "glucose toxicity".

In many cases, type 2 diabetes progresses without any subjective symptom, and a serious complication will develop if diabetes is left as it is.

Terribly, diabetes develops complications of peculiar angiopathy and neuropathy. Such complications occur when blood glucose control has not been satisfactory during progress of disease for a long period, such as 5 years, 10 years or 20 years.

For example, diabetic retinopathy and cataract, which are typical maladies, cause vision disorder, and nephropathy causes proteinuria, swelling, and in course of time, leads to uremia. Neoropathy such as feeling of numbness in hands and legs and nerve pain may develop all over the body. Diabetes also accelerates arteriosclerosis, causing angina pectoris, myocardial infarction, cerebral apoplexy and cerebral thrombosis direct to the cause of death.

Therefore, primary objects of treatment of diabetes are to prevent the complications and to inhibit the progress. In order to prevent complications, control of blood glucose is a very important factor.

For the treatment of type 2 diabetes, dietary therapy and exercise therapy are performed, which is intended to normalize blood glucose. However, when the above two treatments are not sufficient to normalize blood glucose, oral medicament or insulin injection are employed as medical treatment, so that blood glucose is desirably controlled.

Medicaments used for the treatment of diabetes are as follows:
(1) "sulfonylurea (SU) type drug" acting on pancreatic *β* cells for promoting secretion of insulin;
(2) "biguanide (BG) type drug" acting mainly on the liver for elevating glucose disposal capacity in the liver and inhibiting release of glucose from the liver;
(3) " α -glucosidase inhibitors (AGI)" for depressing hyperglycemia after meals by inhibiting α -glucosidase (disaccharide hydrolysate enzyme) in the intestinal tract and holding up absorption of glucose through the intestinal tract;
(4) "insulin sensitizer (Thiazolidinedione, TZD)" for assisting a decrease of blood glucose by promoting the effects of insulin in the cells and reducing insulin resistance; and
(5) Insulin preparation.

The most suitable treatment program combining the dietary therapy, exercise therapy, and medication is prepared for controlling blood glucose depending on the state of the individual diabetic patients.

However, the treatment program largely depends on knowledge and empirical rule of specialists, with no ready-made program.

On analyzing the knowledge/empirical rule of medical specialists of diabetes, the treatment policy and program are settled based on detailed understanding of the pathophysiologic condition of the individual diabetic patients from clinical findings, laboratory test results and the like.

For example, based on such clinical findings and laboratory results, when the pathophysiologic condition of a diabetic patient is classified from four factors of "excessive hepatic glucose production", "insulin secretion ability", "insulin resistance", and "glucose toxicity " which qualifies these factors, the pathophysiologic condition of diabetes is classified as follows.
A. Type 1 diabetes
B. Type 2 diabetes (peripheral insulin resistance)
   Utilization of glucose in muscles or peripheries is lowered. Most of the patients are obese.
C. Type 2 diabetes (excessive hepatic glucose production)
   The promotion effect of hepatic glycogen synthesis and inhibitory action of gluconeogenesis are lowered. Even though patients are not obese, visceral fat is accumulated in many cases.
D. Type 2 diabetes (glucose toxicity)
   Excessive intake of glucose is continued, and thus continuous hyperglycemic condition develops. "Soft drink Ketosis" is included.
E. Type 2 diabetes (decrease of insulin secretion)

Secretion of insulin is incomplete because of exhausted pancreatic *β* cells. Patients are not obese, but are rather emaciated.

It is difficult to obtain a satisfactory control of blood glucose by a standardized treatment program for such a pathophysiologic condition, and it is required to provide the most suitable treatment program in combination of meals, exercises, and medications depending on the individual pathophysiologic conditions.

On the other hand, by general practitioners or general internists who are not specialists of diabetes, the most suitable treatment programs for individual diabetic patients are not necessarily performed, sometimes leading to a undesirable control of blood glucose.

Therefore, if knowledge and empirical rule of specialists can be used as diagnosis support information for general practitioners or general internists who are not specialists of diabetes, in any form, it will be a great help for diabetic patients.

Hitherto, there exist some types of diagnosis support system for diabetes. However, many of them are such simple systems as monitoring the measurements of the patient's blood glucose level, or determining dosage of insulin from the measurements such as the patient's blood glucose level. These support systems do not give satisfactory support information to doctors who are not specialists (For example, see Japanese Unexamined Patent Publication No. Hei 10 (1998)-332704 and Japanese Unexamined Patent Publication No. Hei 11 (1999)-296598).

The well known Diagnostic Criteria for diabetes is defined by Japan Diabetic Society in 1999. It helps a diagnosis of diabetes by classifying patients into categories of "normal type", "borderline diabetic type" and "diabetic type", based on presence or absence of typical symptom of diabetes and the results of the oral glucose tolerance test, and patients who were judged as "diabetic type" twice as a result of such medical inspection are diagnosed as "diabetic patients".

Existing computer systems that implement the diagnosis support for diabetes, in many cases, automate the determination based on such diagnostic criteria. For example, when the user enters the result of the oral glucose tolerance test into the computer system, the computer automatically compares the input data and the predetermined standard value, and the supplies the result as to which one of "normal type", "borderline diabetic type", and "diabetic type" the patient is applied to.

There exists a further advanced system which has an additional function to judge whether or not the patient is obese when the patient's height and weight are entered in to the computer system, and then to determine automatically medicaments to be administered.

In the system of the related art, the subjects are classified into "patients having normal glucose tolerance", "patients having impaired glucose tolerance", and "diabetic patients". However, this system does not estimate etiology based on the aforementioned four factors such as glucose toxicity and the others.

In addition, in the system of the related art, although the current pathophysiologic condition of patients can be classified, the diagnosis support process cannot be performed by given times at given intervals. Therefore, a change in the patient condition over an elapsing time or in the process of medical consultation cannot be figured out in detail.

In order to grasp precisely the change in condition of the patient, an advanced experience and subjective diagnosis of the specialists are necessary, and thus doctors who are not specialists of diabetes or doctors who are not much experienced in diagnosis of diabetes cannot diagnose correctly under the present situation. In addition, since such doctors who are not specialists of diabetes are not sufficiently trained with standardized knowledge on what kind of inspection is required or what is an etiology considerable from the results of the inspection, diagnosis varies between doctors, and thus the most suitable treatment is difficult to find.

### SUMMARY OF THE INVENTION

In view of such circumstances, an object of the invention is to provide a diagnostic system for diabetes that helps to estimate and analyze etiology from the entered clinical testing data, and figure out the change of conditions of the patient for providing support information which is helpful for diagnosis even to doctors who are not specialists of diabetes.

The invention provides a diagnosis support system for diabetes including: a diagnostic data input unit for entering diagnostic data including clinical testing data and clinical findings of a patient; a pathophysiologic condition pattern analyzing unit for analyzing the pathophysiologic condition of diabetes of the patient by comparing diagnostic data and predetermined criteria of analysis; a diagnosis support information generating unit for generating diagnosis support information based on the diagnostic data and criteria of diagnosis predetermined for each analyzed pathophysiologic condition, and a diagnosis support information output unit for outputting information obtained by the pathophysiologic condition pattern analyzing unit and the diagnostic information generating unit.

With this system, objective and quantitative support information for diagnosing diabetes can be obtained, and thus even medical doctors who are not specialists of diabetes can give diagnosis and treatment at the same level as the specialist or at least at the level close thereto objectively with precision.

### BRIEF DESCRIPTION OF THE DRAWINGS

Fig. 1 is a block diagram showing a general construction of a diagnosis support system for diabetes according to an embodiment of the invention;
Fig. 2 is a schematic flowchart showing a general process of the diagnosis support system for diabetes according to an embodiment of the invention;
Fig. 3 is a flowchart of a process of determining peripheral insulin resistance according to an embodiment of the invention;
Fig. 4 is a flowchart of a process of determining hepatic .glucose production according to an embodiment of the invention;
Fig. 5 is a flowchart of a process of determining glucose toxicity as a result of being subjected to hyperglycemia for a long time according to an embodiment of the invention;
Fig. 6 is a flowchart of a process of determining decrease of insulin secretion according to an embodiment of the invention;
Fig. 7 is a flow chart of a process of generating diagnosis support information according to an embodiment of the invention;
Fig. 8 is a flowchart of a process of deciding a treatment policy for peripheral insulin resistance according to an embodiment of the invention;
Fig. 9 is a flowchart of a process of deciding a treatment policy for excessive hepatic glucose production according to an embodiment of the invention;
Fig. 10 is a flowchart of a process of deciding a treatment policy for glucose toxicity as a result of being subjected to hyperglycemia for a long time according to an embodiment of the invention.
Fig. 11 is a flowchart of a process of deciding a treatment policy for decrease of insulin secretion according to an embodiment of the invention.
Fig. 12 is an explanatory drawing showing an embodiment of the main evaluation values to be entered in the system of the invention and an embodiment of the result (score) of the pathophysiologic condition pattern analyzing process.
Fig. 13 is an explanatory drawing of the pathophysiologic conditions of diabetes classified by the scores obtained from three etiologies of "insulin resistance", "glucose toxicity", and "decrease of insulin secretion".
Fig. 14 is an explanatory drawing of the pathophysiologic conditions of diabetes classified by the scores obtained from three etiologies of "insulin resistance", "excessive hepatic glucose production", and "decrease of insulin secretion".

### DESCRIPTION OF THE PREFERRED EMBODIMENTS

The invention provides a diagnosis support system for diabetes including: a diagnostic data input unit for entering diagnostic data including clinical testing data and clinical findings of a patient; a pathophysiologic condition pattern analyzing unit for analyzing the pathophysiologic condition of diabetes of the patient by comparing diagnostic data and predetermined criteria of analysis; a diagnosis support information generating unit for generating diagnosis support information based on the diagnostic data and criteria of diagnosis predetermined for each analyzed pathophysiologic condition, and a diagnosis support information output unit for outputting information obtained by the pathophysiologic condition pattern analyzing unit and the diagnostic information generating unit.

Preferably, the criteria of analysis and criteria of diagnosis are stored in a non-volatile semiconductor storage element such as ROM or PROM, or in a non-volatile storage device such as a hard disk, so that they can be added, deleted, or modified as needed. Function of each functional block such as the pathophysiologic condition pattern analyzing unit is realized by the cooperative operation of the hardware and the program.

The pathophysical condition pattern analyzing unit may be constructed to include the criteria of analysis including determination of insulin resistance, determination of hepatic glucose production, determination of glucose toxicity as a result of being subjected to hyperglycemia for a long time, and determination of decrease of insulin secretion, calculate evaluation values obtained from each criterion of analysis, and compare the obtained evaluation values so that the pathophysiologic condition of diabetes is analyzed.

The diagnosis support information generating unit may be constructed to generate diagnosis support information for treatment of the patient using the criteria of diagnosis including a standard of treatment policy for the patient whose evaluation value of peripheral insulin resistance is the largest, a standard of treatment policy for the patient whose evaluation value of excessive hepatic glucose production is the largest, a standard of treatment policy for the patient whose evaluation value of glucose toxicity as a result of being subjected to hyperglycemia for a long time is the largest, and a standard of treatment policy for the patient whose evaluation value of decrease of insulin secretion is the largest.

Furthermore, the diagnosis support information formed by the diagnosis support information generating unit may include information on the analyzed pathophysiologic condition including the evaluation value and information on exercise therapy, dietetic therapy, and medicinal treatment.

The diagnosis support system for diabetes of the invention may further include a biomodel generating unit for generating a biomodel by estimating a patient-specific biological parameter of diabetes using the entered diagnostic data and information on the pathophysiologic condition analyzed by the pathophysiologic condition pattern analyzing unit, and a pathophysiologic condition simulation unit for estimating the pathophysiologic condition after treatment by giving the generated biomodel a predetermined treatment based on a virtual treatment policy in a simulating manner.

The invention also provides a diagnosis support program for diabetes for allowing a computer to implement a diagnostic data input function for allowing input of diagnostic data including clinical testing data and clinical findings of a patient, a pathophysiologic condition pattern analyzing function for analyzing the pathophysiologic condition of diabetes of the patient by comparing the diagnostic data and predetermined criteria of analysis, a diagnosis support information generating function for generating diagnosis support information by using the diagnostic data and criteria of diagnosis predetermined for each analyzed pathophysiologic condition, and a diagnosis support information output function for outputting information obtained by the pathophysiologic condition pattern analyzing function and the diagnostic information generating function.

The invention also provides a diagnosis method of a diagnosis support system for diabetes comprising: a diagnostic data input step for entering diagnostic data including clinical testing data and clinical findings of a patient; a pathophysiologic condition pattern analyzing step for analyzing the pathophysiologic condition of diabetes of the patient by comparing the diagnostic data and predetermined criteria of analysis; a diagnosis support information generating step for generating diagnosis support information based on the diagnostic data and criteria of diagnosis predetermined for each analyzed pathophysiologic condition, and a diagnosis support information output step for outputting information obtained by the pathophysiologic condition pattern analyzing step and the diagnostic information generating step.

Referring now to the drawings, the invention will be described based on the embodiment shown in the attached drawings, however, the invention is not limited thereto.

### <System Configuration>

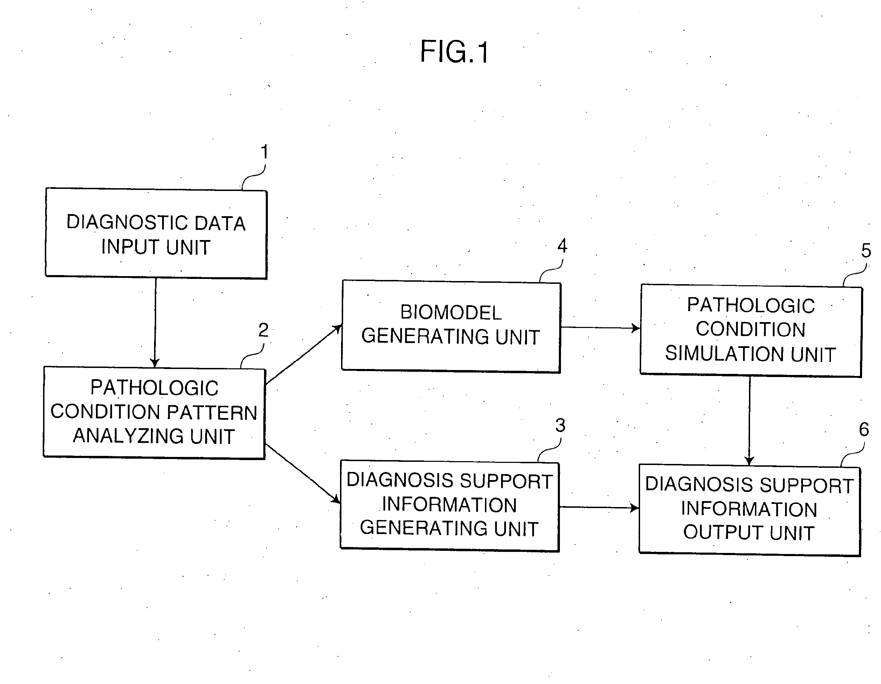

Fig. 1 is a block diagram showing a general construction of a diagnosis support system for diabetes according to an embodiment of the invention.

As shown in Fig. 1, the diagnosis support system for diabetes according to the invention includes a diagnostic data input unit 1, a pathophysiologic condition pattern analyzing unit 2, a diagnosis support information generating unit 3, a biomodel generating unit 4, a pathophysiologic condition simulation unit 5, and a diagnosis support information output unit 6.

These components may be implemented by namely a microcomputer including a CPU, a ROM, a RAM, a timer, an I/O controller, and so on. Functions of the respective components are achieved by the CPU, which reads out a program stored in the ROM, the RAM or the hard disk to the main storage and performing a predetermined process based on the program.

The diagnostic data input unit 1 is an unit for entering values of clinical testings of blood glucose level and so on, information on findings obtained from doctor's question, various information which is entered in advance to a database or the like into the present system. For example, it is a unit corresponding to various input devices such as a keyboard, an OCR, a scanner, a card reader, and a mouse.

Entered information is stored in a non-volatile storage device such as a hard disk so as to be used, for example, by the pathophysiologic condition pattern analyzing unit 2.

In the invention, diagnostic data to be entered may be at least as follows. However, it is not limited thereto, and other evaluation values may be entered as needed.

Such data includes fasting insulin value "a" (µu/ml), blood glucose level "b" (mg/dl), HOMA-IR value (=a ×b/405), insulin OGTT highest value (µU/ml), quantitative 24 hours urine C-peptide (µg), glycated hemoglobin index HbA_{1c}, presence of reduction of weight, BMI value, ΔIRI/ΔBS, ketones in urine (qualitative), and so on.

The clinical findings include the state of obesity, the state of fasting and postprandial blood glucose level, the state of dietary intake of carbohydrate.

The pathophysiologic condition pattern analyzing unit 2 is a unit for analyzing the pathophysiologic condition of diabetes of the patient based on the entered diagnostic data.

In an embodiment shown below, the etiologies causing diabetes is classified into the following four factors.
(a) peripheral insulin resistance
(b) excessive hepatic glucose production
(c) glucose toxicity as a result of being subjected to hyperglycemia for a long time
(d) decrease of insulin secretion

In the pathophysiologic condition pattern analyzing unit 2, evaluation values relating these four etiologies are calculated respectively. The evaluation values means the degree of incidence of the etiology to diabetes. Each evaluation value is calculated based on the criteria of analysis as described below, and output as a real value. The evaluation value is referred to as score, hereinafter.

The criteria of analysis are stored in the hard disk in advance, and the scores are calculated by comparing the input data and the criteria in sequence based on the predetermined analysis processing program.

The calculated score is given to the diagnosis support information generating unit 3, and used for generating support information such as a method of treatment.

The diagnosis information generating unit 3 is a unit for generating support information such as a method of treatment which is considered to be most suitable based on the criteria of diagnosis as will be described later, using the result of analysis in the pathophysiologic condition pattern analyzing unit 2 and a database in which diagnostic data entered from the diagnostic data input unit 1 and technical know-how of specialists are stored.

The database in which the technical know-how of specialists are stored includes knowledge relating to medicament for diabetes, knowledge relating to exercise therapy, and knowledge about dietary therapy of specialists, and is systemized as a treatment policy according to the pattern of each pathophysiologic condition, clinical findings or experience of surgical operation of the patient. Such information is stored in the storage device such as a hard disk or the like.

Knowledge of medicament includes knowledge relating to candidates of medicament that can be given depending on the patholophysiologic condition, selecting order or dosage of medicament depending on the clinical findings, types of medicament that cannot be given to the patient depending on the condition of the patient (information on contraindication). The ratio of medicaments to be given may be determined by functions of strength of parameter such as the degree of incidence of the respective etiologies to diabetes in the specific patient, or whether or not the specific patient has any abnormality in internal organ like kidney.

For example, such policy of medication that TZD and AGI are to be administered together is based on fulfillment of conditions such that increase in insulin resistance has a largest impact on the etiologies of diabetes of the specific patient, the patient does not develop symptoms of cardiac failure, no electrolyte imbalance is found, the patient has no experience of surgical operation in his/her digestive tract, and so on.

Knowledge of exercise therapy includes knowledge relating to strength of exercise, the amount of exercise, and recommended types of exercise depending on the pathophysiologic conditions.

Knowledge of dietary therapy includes knowledge relating to allowable intake calories, allowable intake amounts of the respective nutritional elements depending on the pathophysiologic condition.

The biomodel generating unit 4 is a unit for estimating patient-specific biological parameters and generating a biomodel based on the entered diagnostic data and the result of analysis performed by the pathophysiologic condition pattern analyzing unit 2.

The biological parameter means, for example, "utilization of glucose", "insulin secretion", "hepatic glucose production". These parameters are supplied to the pathophysiologic condition simulation unit 5, and are used for estimating the pathophysiologic condition of the patient in the future. For example, "utilization of glucose" means the amount of consumption of glucose in each cell of the body tissues such as muscles or adipose tissues, and is used for estimating insulin resistance.

The pathophysiologic condition simulation unit 5 is a unitfor estimating the pathophysiologic condition after a certain treatment is given to the patient using the patient-specific biomodel generated by the biomodel generating unit. 4. For example, in the case in which increase of "insulin secretion" and decrease of "hepatic glucose production" were observed, but increase of "utilization of glucose" was not observed after conduction of a simulation assuming that insulin treatment has given to a patient whose major influential etiology before treatment was glucose toxicity as a result of being subjected to hyperglycemia for a long time, the pathophysiologic condition after treatment is estimated as having the major influential etiology in insulin resistance, and the result representing it is output.

The diagnosis support information output unit 6 is a unit for outputting support information such as a method of treatment generated by the diagnosis support information generating unit 3, and a pathophysiologic condition of a result or treatment estimated by the pathophysiologic condition simulation unit 5.

The diagnosis support information output unit 6 includes a printing apparatus such as a printer, and a display apparatus such as a CRT, an LCD, an EL, and a PDP. Support information is supplied to the doctor and the patient by printing on a predetermined form, or by displaying on a monitor.

Function of each unit such as the pathophysiologic condition pattern analyzing unit 2, the diagnosis support information generating unit 3, the biomodel generating unit 4, and the pathophysiologic condition simulation unit 5 as shown Fig. 1 is realized by the cooperative operation of the hardware and the predetermined program.

The program showing the operating procedure of each functional block is normally stored in a semiconductor element such as a ROM, or a stationary storage device such as a hard disk, and is implemented by being loaded in the main storage of a CPU. However, it may be provided in a form of being stored in various storage media such as a CD-ROM, a MO, a FD, or a DVD-ROM. It is also possible to be provided in a form of being stored in a remote server, and downloaded into the hard disk of the present system via various networks.

### <Processing details of the System>

The processing details of the diagnosis support system for diabetes according to the invention will now be described.

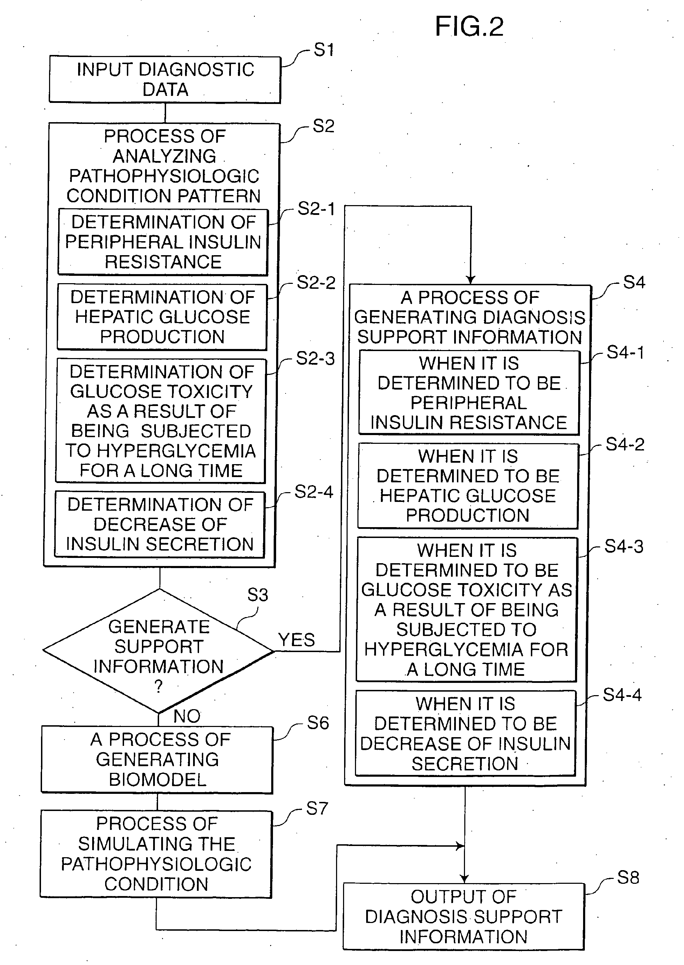

Fig. 2 shows a main flowchart of the diagnosis support system for diabetes of the invention.

In Step S1, diagnostic data of a certain specific patient as described above is entered via the diagnostic data input unit 1.

Then, the pathophysiologic condition pattern analyzing process is activated (Step S2).

In the pathophysiologic condition pattern analyzing process, four determination processes shown below are primarily performed.
Step S2-1: Determination of peripheral insulin resistance
Step S2-2: Determination of hepatic glucose production
Step S2-3: Determination of glucose toxicity
Step S2-4: Determination of decrease of insulin secretion

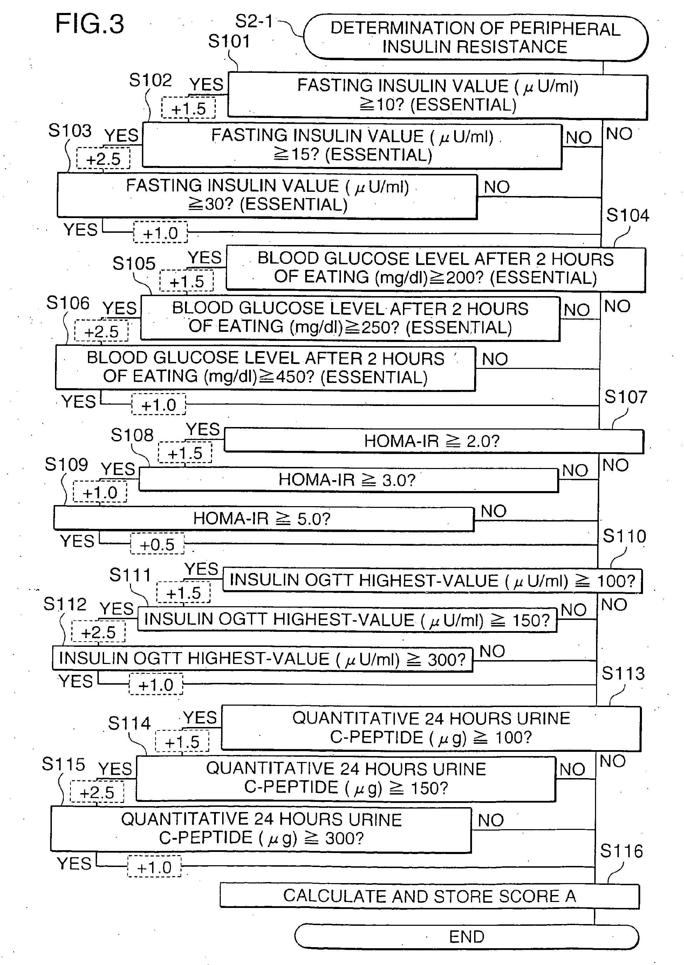

Processing details of each step are shown in Fig. 3 to Fig. 6.

When the determination process of each step is performed, the score of each step is calculated and temporarily stored in a hard disk. For example, in Step S2-1, a process of determining peripheral insulin resistance is performed, and an evaluation value (referred to as score A) representing how much extent insulin resistance affects as a cause of diabetes is obtained. The score A is temporarily stored, and used in a subsequent process of generating diagnosis support information (Step S4).

Likewise, score B, which is an evaluation value of hepatic glucose production is calculated in Step S2-2, a score C representing glucose toxicity is calculated in Step S2-3, and a score D representing decrease of insulin secretion is calculated in Step S2-4.

The larger these scores are, the larger the degree of incidence of the etiologies becomes.

Subsequently, in Step S3, whether the process goes to the process of generating support information (Step S4) or to a process of generating biomodel (Step S6) is determined.

This determination may be performed by prompting the user to input his/her choice by predetermined keystrokes. Alternatively, the user may enter information regarding which process he/she wants the system to perform in Step S1. The determination process in Step S3 is not essential, and may proceed to Step S4, and then sequentially to Steps S6 and S7.

When the process of generating support information is not performed in Step S3, the procedure goes to Step S6, where the process of generating biomodel is performed.

In the process of generating biomodel (Step S6), calculation of values of functions based mainly on the entered diagnostic data is performed, and the above-described biological parameters are obtained.

After Step S6, a process of simulating the pathophysiologic condition (Step S7) is performed, and the pathophysiologic condition of the patient after having a treatment is estimated by using the obtained biological parameters. Estimation of the pathophysiologic condition is performed, for example, in such a manner that function values representing the respective biological parameters are calculated by entering such information that "a certain medicament is given by a specified dosage", then, diagnostic data of the specific patient is estimated from increase or decrease of those biological parameters, and then Step S4 is performed using the results.

Subsequently, the procedure goes to Step S8, and information obtained in Steps S2, S6 and S7 which will be useful for diagnosis support are displayed or printed.

When the process of generating support information is performed in Step S3, the procedure goes to Step S4, where the process of generating diagnosis support information is performed. In the process of generating diagnosis support information, any one of four processes shown below is performed depending on the magnitudes of the four stores (A, B, C, D) obtained in Step S2.
Step S4-1: When it is determined to be peripheral insulin resistance
Step S4-2: When it is determined to be hepatic glucose production
Step S4-3: When it is determined to be glucose toxicity as a result of being subjected to hyperglycemia for a long time
Step S4-4: When it is determined to be decrease of insulin secretion

In these four processes, support information including a treatment policy, medicaments to be given, and so on, is generated for each etiology based on a predetermined criteria of diagnosis.

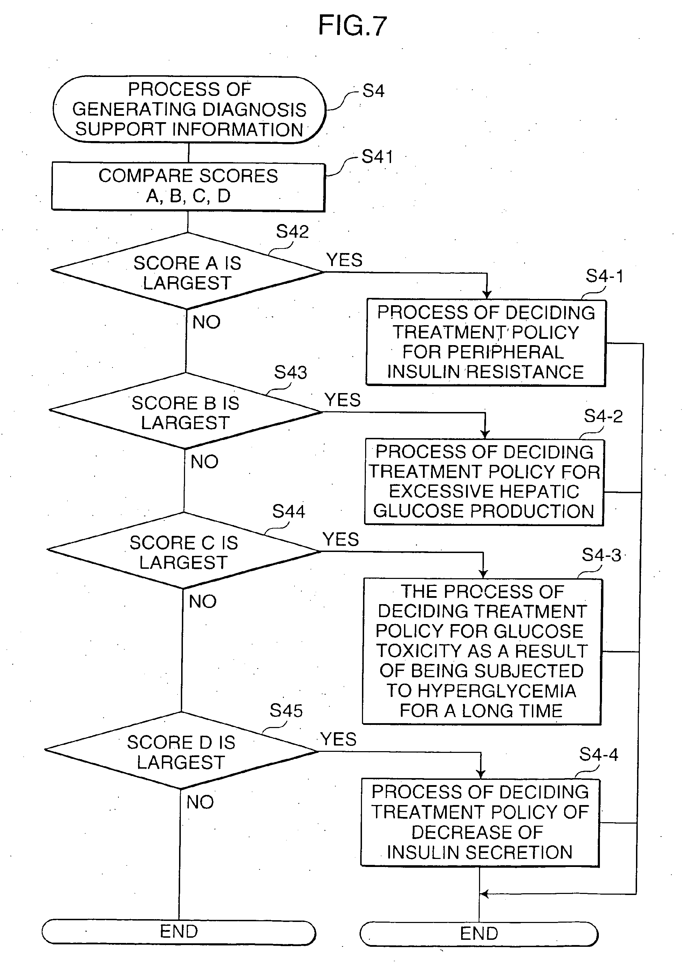

Processing details of each step are shown in Fig. 7 to Fig. 11.

After performing Step S4, generated diagnosis support information will be displayed or printed (Step S8).

The general flow of the diagnosis support system for diabetes according to the invention has been described.

Now, each process of determination in Step S2 will be described.

Fig. 3 shows a flowchart of a process of determining peripheral insulin resistance (S2-1).

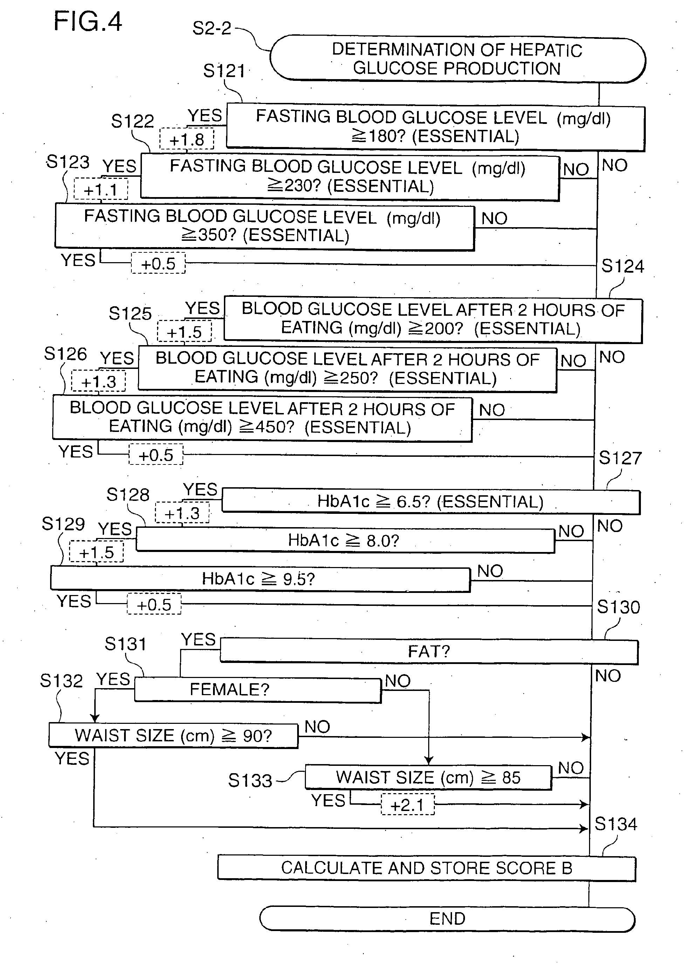

Fig. 4 shows a flowchart of a process of determining hepatic glucose production (S2-2).

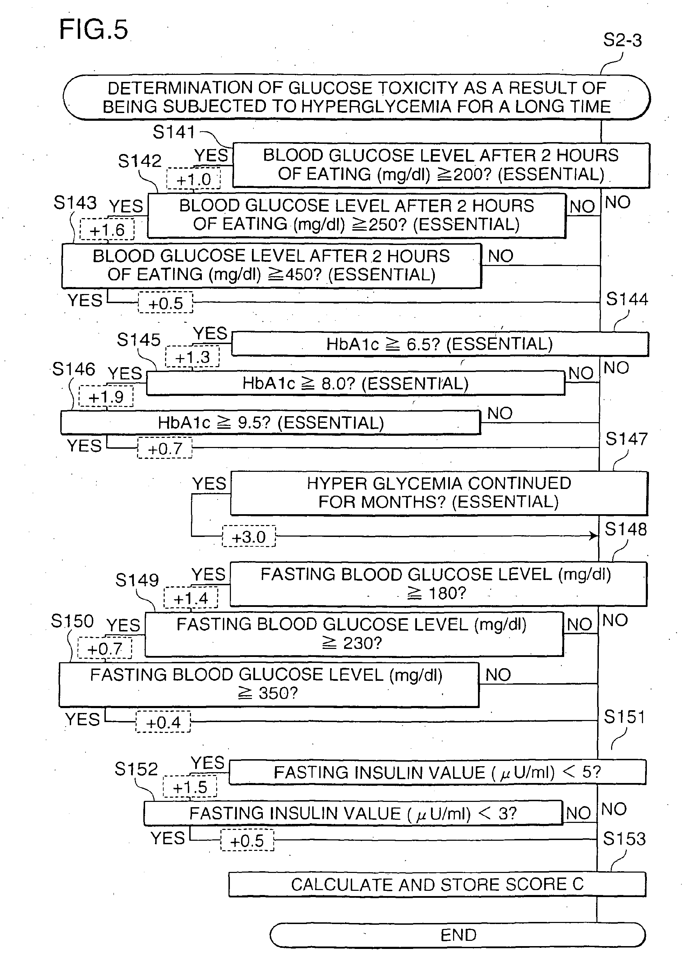

Fig. 5 shows a flowchart of a process of determining glucose toxicity (S2-3).

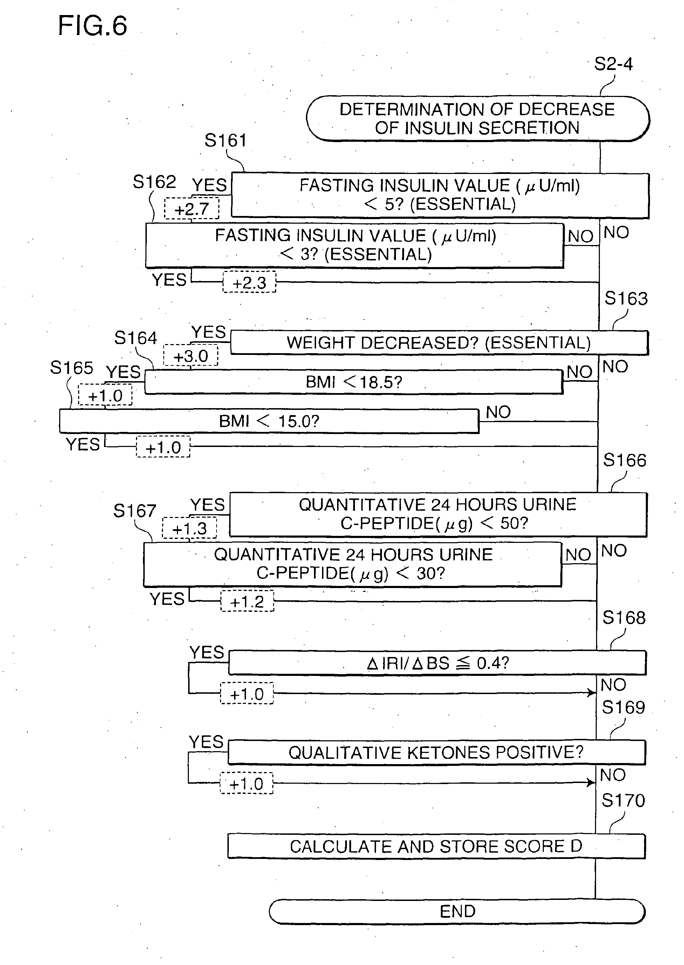

Fig. 6 shows a flowchart of a process of determining decrease of insulin secretion (S2-4).

When the respective processes of Steps S2-1, S2-2, S2-3, and S2-4 are terminated, the score A, B, C, and D are calculated respectively.

Each score (A, B, C, D) is calculated as a summation (SC) of values predetermined in the respective determination process. However, in order to express the score in percentage (%), a value obtained by dividing the calculated value of store SC by a total score SA which is a summation of the highest values, which correspond to the values that will be resulted in the worst case, may be used as a score. That is, Score (A, B, C, D) = (SC/SA) x 100 (%).

When the criteria of analysis includes an inspection K which has not been performed, the total score in this case SA' is obtained by subtracting a value of the score (SB) which must have been obtained in the inspection K from the total score SA of the worst case (SA'=SA-SB).

In this manner, expression in percentage can provide a result that does not depend on the type or the number of conducted inspections.

In the determination process in Fig. 3 (S2-1), each of entered "fasting insulin value", "blood glucose level 2 hours after eating", "HOMA-IR", "insulin OGTT highest-value", "quantitative 24 hours urine C-peptide" is compared with the predetermined value of criterion, respectively.

Here, a variable A for calculating the score A is provided. The initial value is zero. When the result of each determination from Steps S101 to S115 is "YES", predetermined scores are added to the variable A.

For example, in Step S101, when the entered "insulin value on an empty stomach" is 10 or higher, it is determined to be "YES", and a value 1.5 is added to the variable A.

When the inspection of "HOMA-IR" is not conducted, it is determined to be "NO" in step S107, and thus no value is added and the procedure goes to the next determination (S110).

After each determination from Step S101 to Step S115 is completed, the score A is calculated and stored using the value of the variable A in Step S116.

The score A may be the value of the variable A as is. However, as described above, it is preferable to employ a percentage expression (variable A/SA) × 100 which is obtained by dividing the variable A by a total score SA (=23 points), which is a total value when the determination followed a route which brings about the highest score in the entire step S2-1.

Likewise, in Step S2-2 in Fig. 4, values are added to a variable B, which is initially zero, at every determination. After Steps S121 to S133 are terminated, the score B is calculated from the variable B in Step S134.

In Step S2-3 in Fig. 5, values obtained at every determination at S141 to S152 are added in sequence to a variable C, which is initially zero. After Steps S141 to S152 are terminated, a score C is calculated from the variable C in Step S153.

Furthermore, in Step S2-4 in Fig. 6, values obtained at every determination at S161 to S169 are added in sequence to a variable D, which is initially zero. Then, the score D is calculated from the variable D in Step S170. In this manner, processing in Step S2 is terminated and the scores A, B, C, and D are calculated.

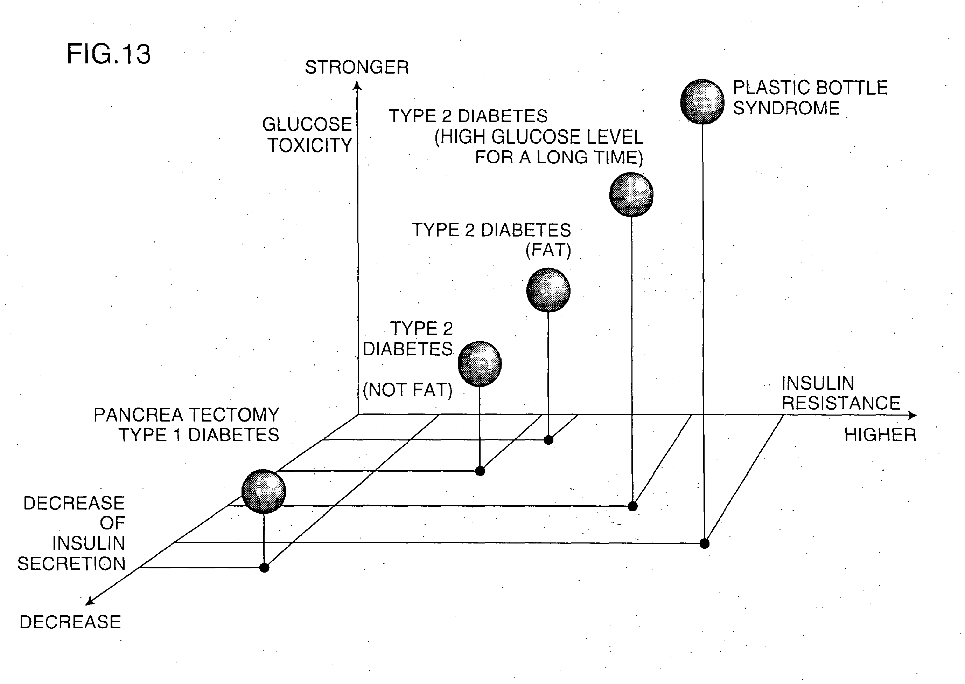

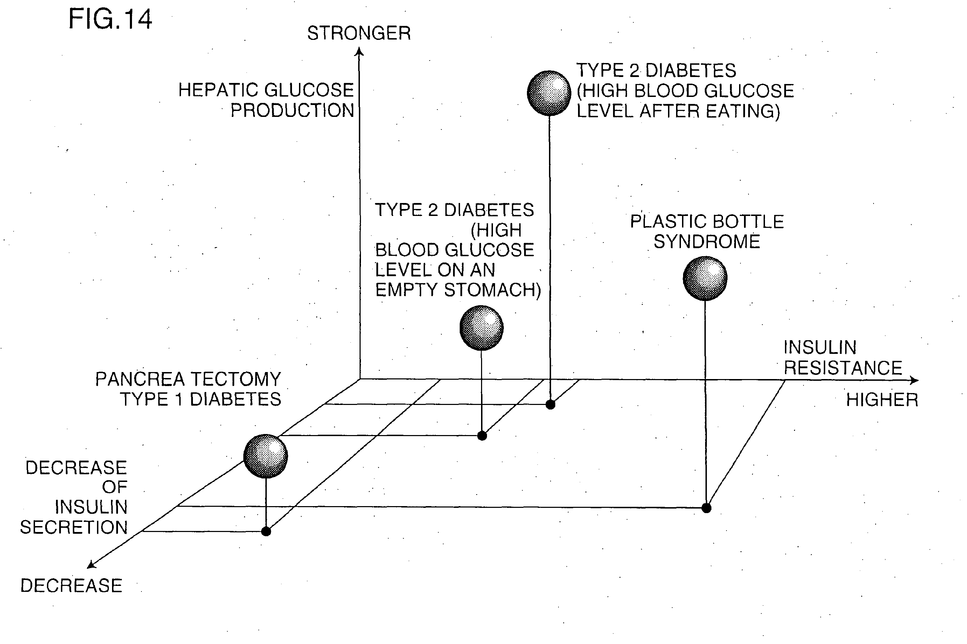

In this manner, the four evaluation values (scores) are obtained by the four determination processes (Fig. 3 to Fig. 6) of the process of analyzing pathophyciologic condition (Step S2). Then, by comparing the values of these scores, which etiology is highest in the amount of incidence may be determined. In addition, by performing classification process based on the combination of absolute magnitudes of each of four scores, the pathophysiologic condition of the patient may be determined which one of the above-described five pathophysiologic conditions of diabetes it belongs to. For example, as shown in Fig. 13, when three scores of "insulin resistance", "glucose toxicity", and "decrease of insulin secretion" are mapped in a three-dimensional space, they are plotted to a different positions by a clinical features, and thus the pathophysiologic conditions may be classified according to the primary etiology. As shown in Fig. 14, when three scores of "insulin resistance", "hepatic glucose production", and "decrease of insulin secretion" are mapped in a three-dimensional space, they are plotted to a different positions by a clinical features, and thus the pathophysiologic conditions may be classified according to the primary etiology.

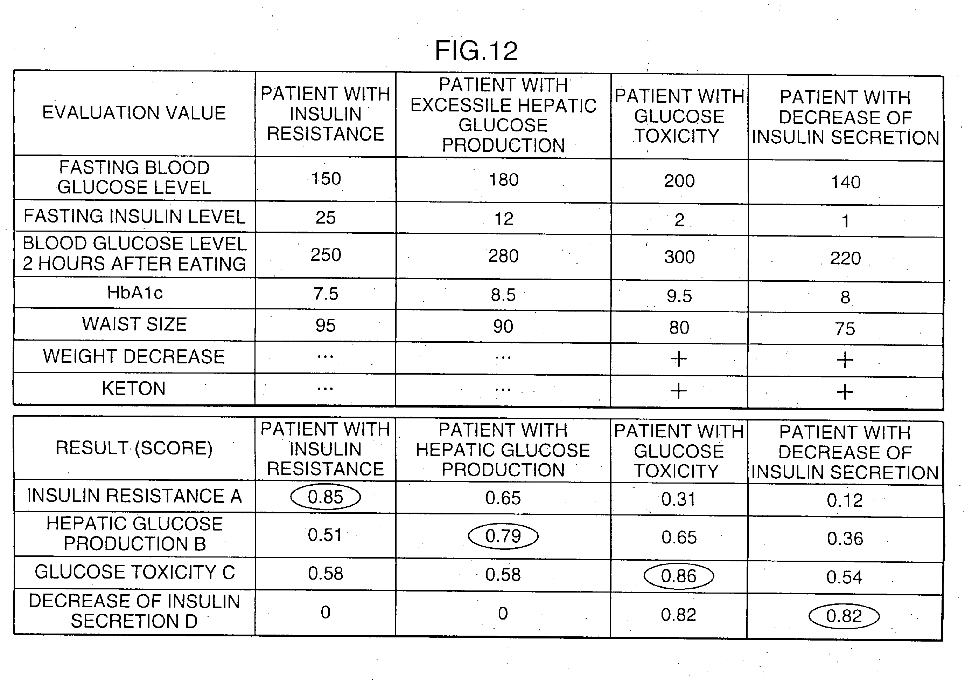

Fig. 12 shows detailed examples of the scores calculated in Step S2 for patients having different etiologies.

For example, in determination of insulin resistance, a patient whose score A is the highest value, 0.85, which represents that insulin resistance is the most influential etiology, is determined as a "insulin resistant patient".

Subsequently, processing details of the process of generating diagnosis support information that is performed by the diagnosis support information generating unit 3 will be described.

Fig. 7 shows a flowchart of the process of generating diagnosis support information (Step S4).

In Step S41, the scores (A, B, C, D) obtained in Step S2 are read and compared. Then, in such comparison, the largest score is searched for out of these four scores. Then a process of deciding treatment policy is performed for the etiology corresponding to the largest score, and the process is terminated.

In Step S42, when the score A is the largest among other scores, the procedure goes to Step S4-1, where "the process of deciding treatment policy for peripheral insulin resistance" is performed.

Likewise, in Step S43, when the score B is the largest, the procedure goes to Step S4-2, where "the process of deciding treatment policy of hepatic glucose production" is performed.

In Step S44, when the score C is the largest, the procedure goes to Step S4-3, where "the process of deciding treatment policy for glucose toxicity is performed.

In Step S45, when the score D is the largest, the procedure goes to Step S4-4, where "the process of deciding treatment policy of decrease of insulin secretion" is performed.

When there are the same scores, it means that a plurality of etiologies are influential. Therefore, in such a case, the process of Step S4 is to be performed for all the scores of the same point, and all the results are to be reflected in such a manner that parallel usage of several types of medicaments, if medication is necessary, are recommended to the patient.

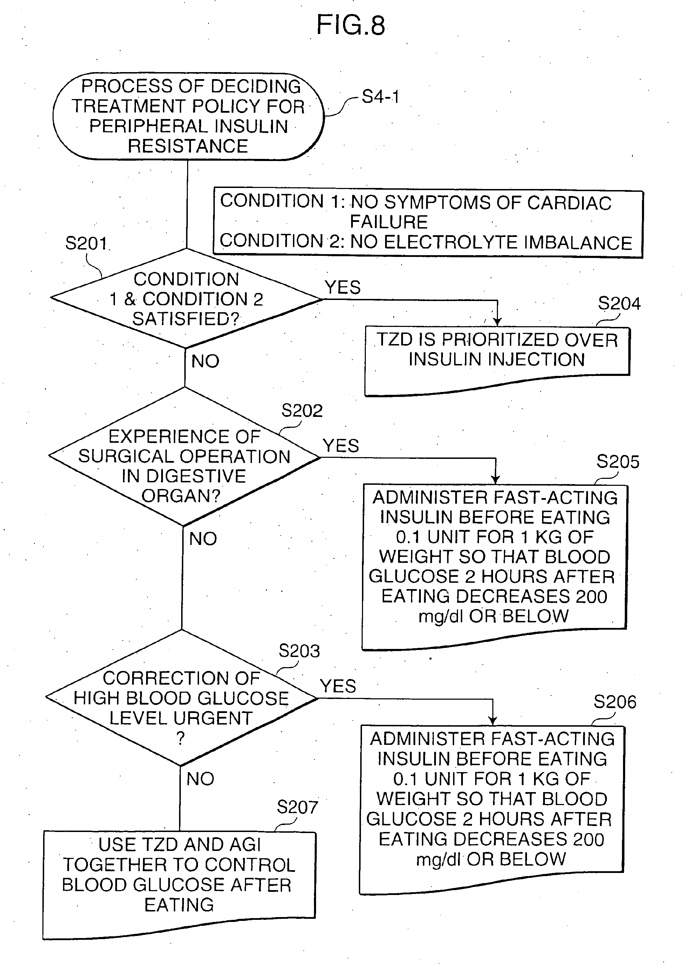

Fig. 8 shows a flowchart of "the process of deciding treatment policy of peripheral insulin resistance" of the Step S4-1.

In Step S201, whether or not "Condition 1: no symptoms of cardiac failure" and "Condition 2: no electrolyte imbalance is found" are satisfied is determined.

Here, the condition "no symptoms of cardiac failure" is determined base on information which is already entered in the database out of entire entered data. The condition "no electrolyte imbalance is found" is determined based on information which is already entered in to the database.

When "Condition 1 and Condition 2 are satisfied, the procedure goes to Step S204, and a treatment policy "TZD is prioritized over insulin injection" is selected.

Step S201, when Condition 1 and Condition 2 are not satisfied, the procedure goes to Step S202, where the supplied information "presence of experience of surgical operation in digestive intestine" is determined. When the patient has no experience of surgical operation, the procedure goes to Step S203, where "whether or not normalization of high blood glucose level should be made as soon as possible" is determined.

Determination relating to normalization of high blood glucose level may be made based on information which is already entered in the database out of entire entered data. In other words, when data saying "normalization of high blood glucose level is urgent" is already entered in the database, it is determined that normalization of high blood glucose level should be made as soon as possible.

In step S202, if the patient has an experience of surgical operation, the policy of treatment in Step S205 is employed.

According to the result of determination in Step S203, the policy of treatment in Step S206 or Step S207 is employed.

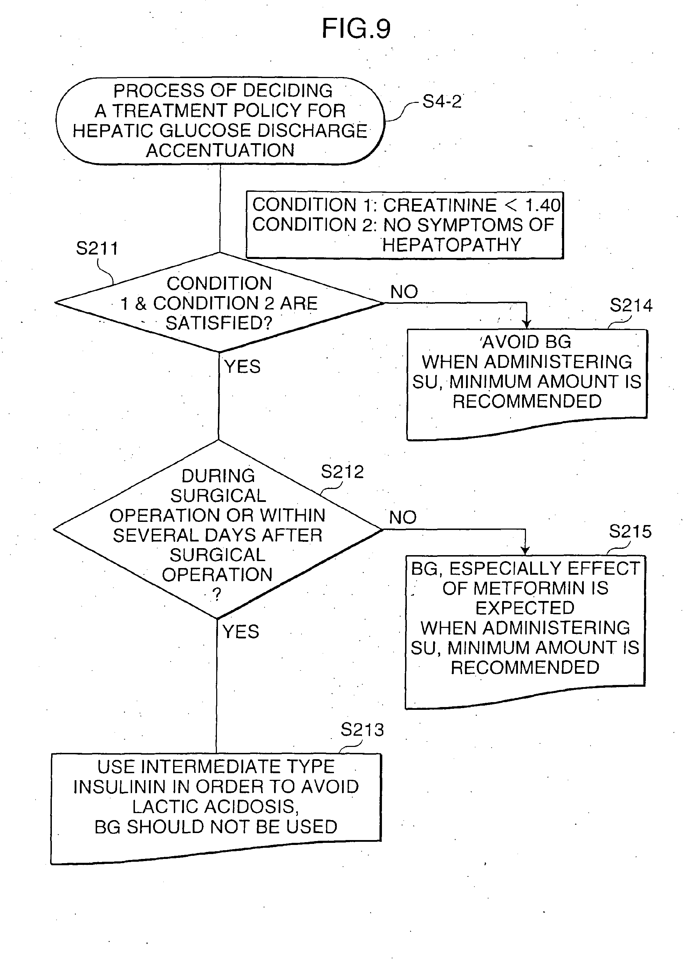

Fig. 9 shows a flowchart of "the process of deciding a treatment policy for hepatic glucose production" of Step S4-2.

In Step S211, whether or not the conditions "Condition 1: creatinine < 1.40" and "Condition 2: no symptoms of hepatopathy" are satisfied is determined.

"Condition 2: no symptoms of hepatopathy" can be determined based on information which is already entered in the database.

"Condition 1 and Condition 2" are not satisfied, the procedure goes to Step S212, where "during surgical operation or within several days after surgical operation?" is determined. Whether the patient is in surgical operation or within several days after surgical operation may be determined from information which is already entered in the database relating the entered patient.

"Several days" is calculated as a function derived from entered diagnostic data regarding the patient.

Based on such determinations, one of the treatment policies shown from S213 to S215 is selected.

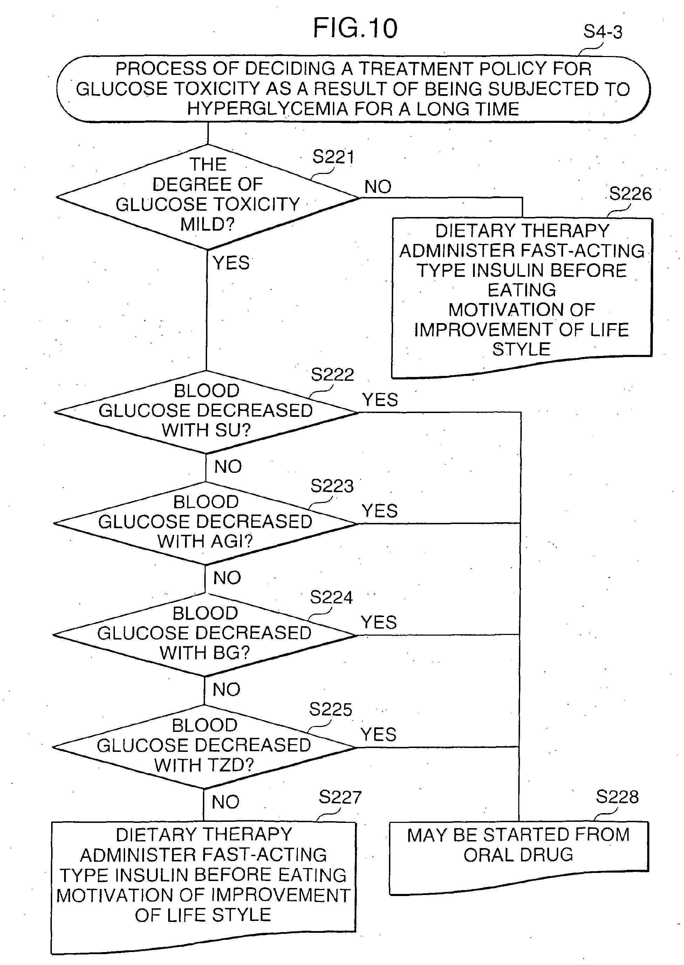

Fig. 10 shows a flowchart of "the process of deciding a treatment policy for glucose toxicity of Step S4-3. In Step 221, "whether or not the degree of glucose toxicity is mild" is determined. Whether or not the glucose toxicity is mild may be determined from the score of glucose toxicity calculated in Steps S2 and S3. For example, when the score of glucose toxicity is the smallest among the scores of four etiologies, it is determined that the degree of glucose toxicity is mild.

When it is determined that the degree of glucose toxicity is not mild, the dietary therapy of Step S226 is employed. In contrast, when the degree of glucose toxicity is mild, the conditions of decrease of blood glucose are determined in Steps S222 to S225 in sequence.

When it is determined that blood glucose is decreased by any one of SU, AGI, BG, and TZD, the treatment policy in Step S228 is employed, and when blood glucose is not decreased by any of those, a treatment policy in Step S227 is employed. Determination of whether or not blood glucose is decreased may be made based on information which is already entered in the database. For example, it is determined that blood glucose is decreased when the patient has already administered with a certain medicament and blood glucose was decreased according to data entered after administration of the medicament.

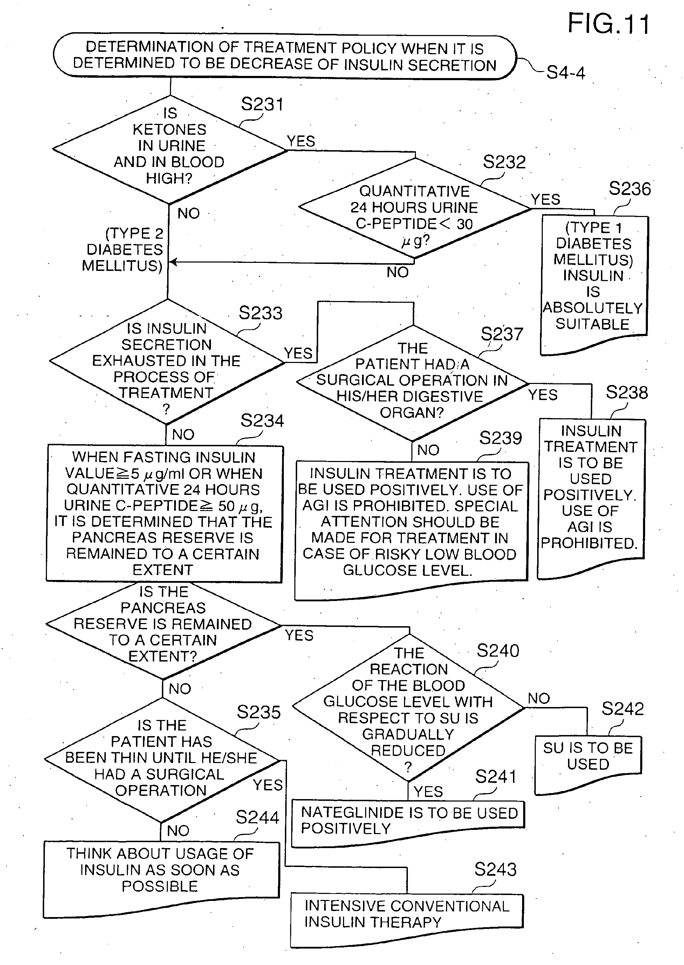

Fig. 11 shows a flowchart of "a process of deciding a treatment policy for decrease of insulin secretion" of Step S4-4.

In step S231, whether or not "the level of ketones in urine and in blood is high" is determined. The term "high" means, for example, the level of ketones in urine in entered data is in the order of +2 or higher.

When it is determined that the level of ketones is high, the procedure goes to Step S232, where whether or not the "quantitative 24 hours urine C-peptide" in entered data is smaller than 30 µg is determined. When the value is smaller than 30 µg, it is determined to be "Type 1 diabetes". Therefore, the treatment policy shown in Step S236 is employed.

When it is determined to be "NO" in Step S231 or S232, the procedure goes to Step S233, where "whether or not insulin secretion is exhausted in the process of treatment" is determined. Whether or not it is exhausted can be determined by fasting insulin level or quantitative 24 hours urine C-peptide. For example, when the value of quantitative 24 hours urine C-peptide is lower than 50 µg, it is determined that insulin secretion is exhausted.

When it is determined to be exhausted, the procedure goes to Step S237, and whether or not the patient had a surgical operation in his/her digestive organ is determined. Based on the result, a treatment policy as in Step S238 or S239 corresponding to such determination is employed.

When it is determined that it is not exhausted in Step S233, the procedure goes to Step S234. When it is determined that a pancreas reserve is remained to a certain extent in Step S234, the procedure goes to Step S240, and if not, the procedure goes to Step S235.

Whether or not the pancreas reserve is remained to a certain extent may be determined by whether or not one of the following conditions is satisfied:
(1) fasting insulin value ≥ 5 µg/ml
(2) quantitative 24 hours urine C-peptide ≥ 50 µg.
In other words, when one of these conditions is satisfied, it is determined that the pancreas reserve is remained to a certain extent.

In step S240, whether or not the reaction of the blood glucose level with respect to SU is gradually reduced is determined. Based on the result of determination, a treatment policy as in Step S241 or S242 is employed. Determination whether or not the reaction of the blood glucose level is reduced may be made, for example, by using information which is already entered in the database. In this case, when the blood glucose level after SU administration is used is not decreased with respect to the level before using SU, it is determined that the blood glucose level does not react on SU.

In Step S235, whether or not the patient has been thin until he/she had a surgical operation is determined. Based on the result of determination, a treatment policy as in Step S243 or S244 is employed. Whether or not the patients has been thin until he/ she had a surgical operation may be determined by information which is already entered in the database, and when the state of BMI ( = weight × 10000/(height × height)) < 18 was repeated by a plurality of times, it is determined that the patient has been thin until he/she had a surgical operation.

The detailed flowchart of the process of generating diagnosis support information has been described thus far. However, it is not limited thereto, and the criteria of diagnosis may be added, deleted, or modified as needed considering the condition of the patient, a unique criteria of the doctor, development of study of diabetes, such as research paper.

It is also possible to employ a specific tool for adding or modifying the criteria of diagnosis so that the user of the present system or the specialist can add or delete the criteria of diagnosis easily.

After the treatment policy is decided as described above, diagnosis support information including the decided treatment policy is presented to the doctor in Step S8 as described above. Since the digitalized results of analysis (scores) may be obtained and the diagnosis support information can be presented by repeating the same inspection and analysis at every medical examination for the same patient, change of the condition of the patient over time can be figured out objectively and accurately, and hence a suitable treatment based on a accurate determination over time may be given to the patient.

For example, by observing change in scores, the user is able to know not only the change of the pathophysiologic conditions classified into five categories, but also how the pathophysiologic conditions have changed based on the quantitative scores, so that further suitable determination and treatment are enabled.

With the diagnosis support system of the invention, the pathophysiologic condition can be analyzed and the treatment policy can be established by a standardized criteria of diagnosis which is prepared in advance without depending on experience or subjectivity which is rather unstable. Therefore, even doctors who are not specialists of diabetes can give diagnosis and treatment at the same level as the specialist or at least at the level close thereto objectively with precision.

According to the invention, since the evaluation values which represent the degrees of incidence are obtained for every etiology of diabetes, the pathophysiologic condition of diabetes can be classified quantitatively.

In addition, since diagnosis support information such as the treatment policy is generated based on the standardized criteria of diagnosis, even doctors who are not specialists of diabetes can give diagnosis and treatment to diabetes at the same level as the specialist or at least at the level close thereto quickly.

Furthermore, since the pathophysiologic condition is quantitatively classified, by repeating the same inspection and analysis continuously for the same patient, change of the conditions of the patient can be figured out further objectively and precisely, and thus a suitable diagnosis and treatment can be provided depending on the process over time.

## Claims

1. A diagnosis support system for diabetes comprising:
a diagnostic data input unit for entering diagnostic data including clinical testing data and clinical findings of a patient;
a pathophysiologic condition pattern analyzing unit for analyzing the pathophysiologic condition of diabetes of the patient by comparing the diagnostic data and predetermined criteria of analysis;
a diagnosis support information generating unit for generating diagnosis support information based on the diagnostic data and criteria of diagnosis predetermined for each analyzed pathophysiologic condition, and
a diagnosis support information output unit for outputting information obtained by the pathophysiologic condition pattern analyzing unit and the diagnostic information generating unit.

2. A diagnosis support system for diabetes according to Claim 1, wherein the pathophysiologic condition pattern analyzing unit comprises the criteria of analysis including determination of peripheral insulin resistance, determination of hepatic glucose production, determination of glucose toxicity as a result of being subjected to hyperglycemia for a long time, and determination of decrease of insulin secretion, and analyses the pathophysiologic condition of diabetes by calculating evaluation values obtained from each criterion of analysis, and comparing the obtained evaluation values.

3. A diagnosis support system for diabetes according to Claim 2, wherein the diagnosis support information generating unit generates diagnosis support information for treatment of the patient using the criteria of diagnosis including a standard of treatment policy for the patient whose evaluation value of peripheral insulin resistance is the largest, a standard of treatment policy for the patient whose evaluation value of hepatic glucose production is the largest, a standard of treatment policy for the patient whose evaluation value of glucose toxicity as a result of being subjected to hyperglycemia for a long time is the largest, and a standard of treatment policy for the patient whose evaluation value of decrease of insulin secretion is the largest.

4. A diagnosis support system for diabetes according to Claim 2 or 3, wherein the diagnosis support information generated by the diagnosis support information generating unit includes information on the analyzed pathophysiologic condition including the evaluation value and information on exercise therapy, dietetic therapy, and medicinal treatment.

5. A diagnosis support system for diabetes according to Claim 1, further comprising:
a biomodel generating unit for generating a biomodel by estimating a patient-specific biological parameter of diabetes using the entered diagnostic data and information on the pathophysiologic condition analyzed by the pathophysiologic condition pattern analyzing unit, and
a pathophysiologic condition simulation unit for estimating the pathophysiologic condition after treatment by giving the generated biomodel a predetermined treatment based on a virtual treatment policy in a simulating manner.

6. A diagnosis support program for diabetes for allowing a computer to implement a diagnostic data input function for allowing input of diagnostic data including clinical testing data and clinical findings of a patient, a pathophysiologic condition pattern analyzing function for analyzing the pathophysiologic condition of diabetes of the patient by comparing the diagnostic data and predetermined criteria of analysis, a diagnosis support information generating function for generating diagnosis support information by using the diagnostic data and criteria of diagnosis predetermined for each analyzed pathophysiologic condition, and a diagnosis support information output function for outputting information obtained by the pathophysiologic condition pattern analyzing function and the diagnostic information generating function.

7. A diagnosis support program for diabetes according to Claim 6, wherein the pathophysiologic condition pattern analyzing function comprises the criteria of analysis including determination of peripheral insulin resistance, determination of hepatic glucose production, determination of glucose toxicity as a result of being subjected to hyperglycemia for a long time, and determination of decrease of insulin secretion, and analyses the pathophysiologic condition of diabetes by calculating evaluation value obtained from each criterion of analysis, and analysis, and comparing the obtained evaluation values.

8. A diagnosis support program for diabetes according to Claim 7, wherein the diagnosis support information generating function generates diagnosis support information for treatment of the patient using the criteria of diagnosis including a standard of treatment policy for the patient whose evaluation value of peripheral insulin resistance is the largest, a standard of treatment policy for the patient whose evaluation value of hepatic glucose production is the largest, a standard of treatment policy for the patient whose evaluation value of glucose toxicity as a result of being subjected to hyperglycemia for a long time is the largest, and a standard of treatment policy for the patient whose evaluation value of decrease of insulin secretion is the largest.

9. A diagnosis support program for diabetes according to Claim 7 or 8, wherein the diagnosis support information generated by the diagnosis support information generating function includes information on the analyzed pathophysiologic condition including the evaluation value and information on exercise therapy, dietetic therapy, and medicinal treatment.

10. A diagnosis support program for diabetes according to Claim 6, further comprising:
a biomodel generating function for generating a biomodel by estimating a patient-specific biological parameter of diabetes using the entered diagnostic data and information on the pathophysiologic condition analyzed by the pathophysiologic condition pattern analyzing function, and
a pathophysiologic condition simulation function for estimating the pathophysiologic condition after treatment by giving the generated biomodel a predetermined treatment based on a virtual treatment policy in a simulating manner.

11. A diagnosis method of a diagnosis support system for diabetes comprising:
a diagnostic data input step for entering diagnostic data including clinical testing data and clinical findings of a patient;
a pathophysiologic condition pattern analyzing step for analyzing the pathophysiologic condition of diabetes of the patient by comparing the diagnostic data and predetermined criteria of analysis;
a diagnosis support information generating step for generating diagnosis support information based on the diagnostic data and criteria of diagnosis predetermined for each analyzed pathophysiologic condition, and
a diagnosis support information output step for outputting information obtained by the pathophysiologic condition pattern analyzing step and the diagnostic information generating step.

12. A diagnosis method of a diagnosis support system for diabetes according to Claim 11, wherein the pathophysiologic condition pattern analyzing step comprises the criteria of analysis including determination of peripheral insulin resistance, determination of hepatic glucose production, determination of glucose toxicity as a result of being subjected to hyperglycemia for a long time, and determination of decrease of insulin secretion, and analyses the pathophysiologic condition of diabetes by calculating evaluation values obtained from each criterion of analysis, and comparing the obtained evaluation values.

13. A diagnosis method of a diagnosis support system for diabetes according to Claim 12, wherein the diagnosis support information generating step generates diagnosis support information for treatment of the patient using the criteria of diagnosis including a standard of treatment policy for the patient whose evaluation value of peripheral insulin resistance is the largest, a standard of treatment policy for the patient whose evaluation value of hepatic glucose production is the largest, a standard of treatment policy for the patient whose evaluation value of glucose toxicity as a result of being subjected to hyperglycemia for a long time is the largest, and a standard of treatment policy for the patient whose evaluation value of decrease of insulin secretion is the largest.

14. A diagnosis method of a diagnosis support system for diabetes according to Claim 12 or 13, wherein the diagnosis support information generated by the diagnosis support information generating step includes information on the analyzed pathophysiologic condition including the evaluation value and information on exercise therapy, dietetic therapy, and medicinal treatment.

15. A diagnosis method of a diagnosis support system for diabetes according to Claim 11, further comprising:
a biomodel generating step for generating a biomodel by estimating a patient-specific biological parameter of diabetes using the entered diagnostic data and information on the pathophysiologic condition analyzed by the pathophysiologic condition pattern analyzing step, and
a pathophysiologic condition simulation step for estimating the pathophysiologic condition after treatment by giving the generated biomodel a predetermined treatment based on a virtual treatment policy in a simulating manner.
